# EUROPEAN PATENT SPECIFICATION

(11) **EP 0 969 013 B1**
(45) Date of publication and mention of the grant of the patent: **26.03.2003**
(21) Application number: 98112047.0
(22) Date of filing: 30.06.1998
(51) Int. Cl.: C07K 7/06, C07K 7/08, C07K 14/00, A61K 38/08, A61K 38/10, A61K 38/16

(54) **Peptides for inhibiting HPV E7 proteins**
Peptide zur Inhibition von HPV E7 Proteinen
Péptides destinés a l'inhibition des proteines E7 de HPV

(43) Date of publication of application: 05.01.2000
(73) Proprietor: Deutsches Krebsforschungszentrum Stiftung des öffentlichen Rechts, 69120 Heidelberg (DE)
(72) Inventor: Jansen-Dürr, Pidder, Dr., 6020 Innsbruck (AT); Zwerschke, Werner, Dr., 69115 Heidelberg (DE)
(74) Representative: Schüssler, Andrea, Dr.

(56) References cited:
- EP-A- 0 531 080
- EP-A- 0 666 270
- WO-A-98/20030
- DE-C- 19 649 606
- DATABASE SWISSPROT ID SP4G_BACSU; AC P26937, 1 August 1992 S. CUTTING ET AL.: "Stage IV sporulation Protein FB" XP002090532 & JOURNAL OF MOLECULAR BIOLOGY, vol. 221, 1991, pages 1237-1256,

## Description

The present invention relates to peptides suitable to inhibit HPV E7 proteins, pharmaceutical compositions containing the same as well as the use of both.

As is known, many people suffer from persistent infections caused by human papilloma viruses (hereinafter referred to as HPVs). As is also known, over 95 % of all anogenital carcinomas, particularly carcinomas of uterine cervix, and a considerable percentage of the carcinomas in the oropharynx are associated with persistent infections caused by HPVs. In addition, there are references to the fact that the development and/or manifestation of carcinomas in cells having a persistent infection caused by HPVs requires an uncontrolled expression of HPV genes, particularly of E7. Moreover, it is known from German patent 196 49 606 that HPV E7 proteins bind cellular regulatory proteins and thus may interfere with the cell regulation. Therefore, inhibitors of HPV E7 proteins could represent a possibility of taking steps against HPV-associated diseases.

Thus, it is the object of the present invention to provide a product by means of which HPV E7 proteins can be inhibited.

According to the invention this is achieved by the subject matters defined in the claims.

The European patent application EP 0 666 270 discloses peptides which bind to HPV E7 proteins and inhibit HPV E7 activity.

The applicant has found that HPV E7 proteins bind to short peptides and screened a peptide library (Colas, P. et al., Nature 380, (1996), 548-550) comprising peptide sequences generated randomly, with a "two hybrid" system in which the C-terminal part of an HPV E7 protein, i.e. the part required for the binding to cell regulatory proteins, i.e. AS 39-98 of the HPV16 E7 protein, was used as a screening sample. In this connection, the applicant found that short peptides, particularly the ones listed in Table 1, bind HPV E7 proteins. The applicant has also found that these peptides are suitable to inhibit functions of HPV E7 proteins, e.g. the induction of apoptosis in human osteosarcoma cells U20S, or the stimulation of the growth of E7/2 cells. Reference is made to the below examples.

According to the invention the applicant's findings are used to provide a peptide selected from the peptides listed in Table 1, it being possible that the peptide includes a sequence modification of up to 40 %.

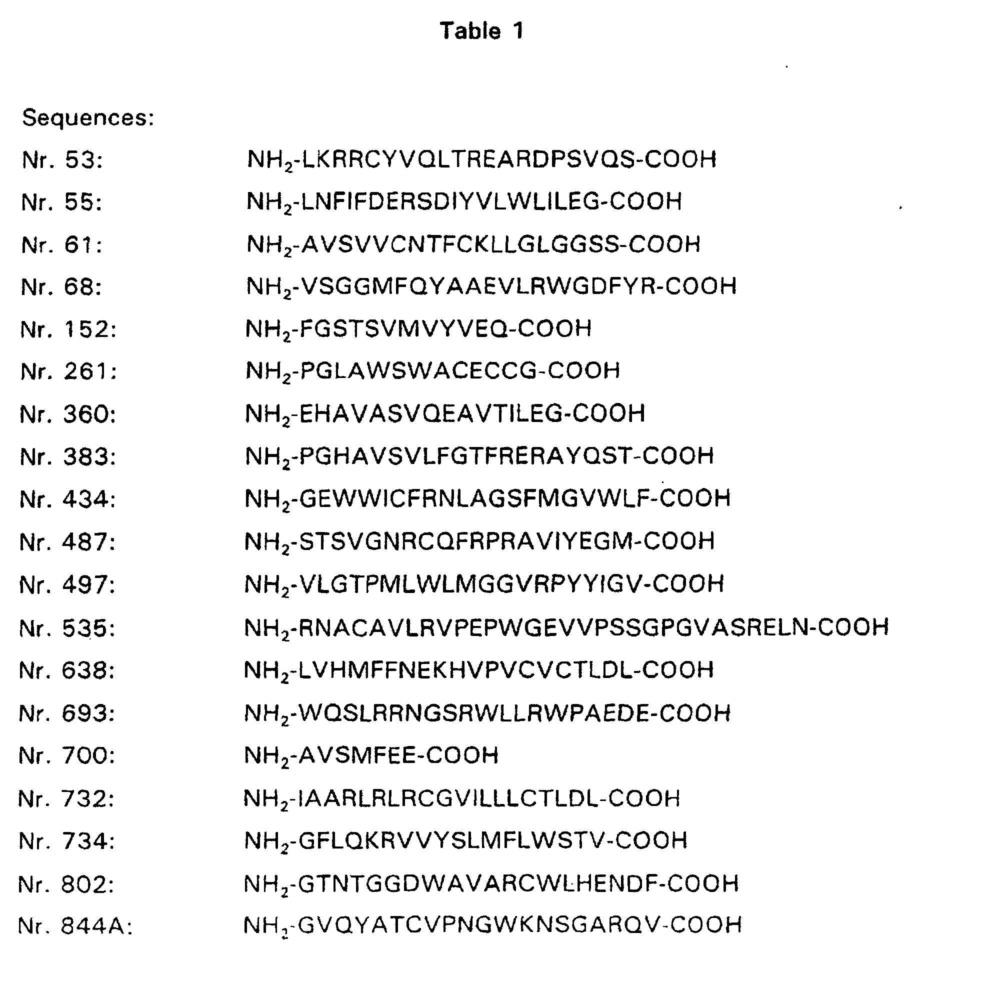

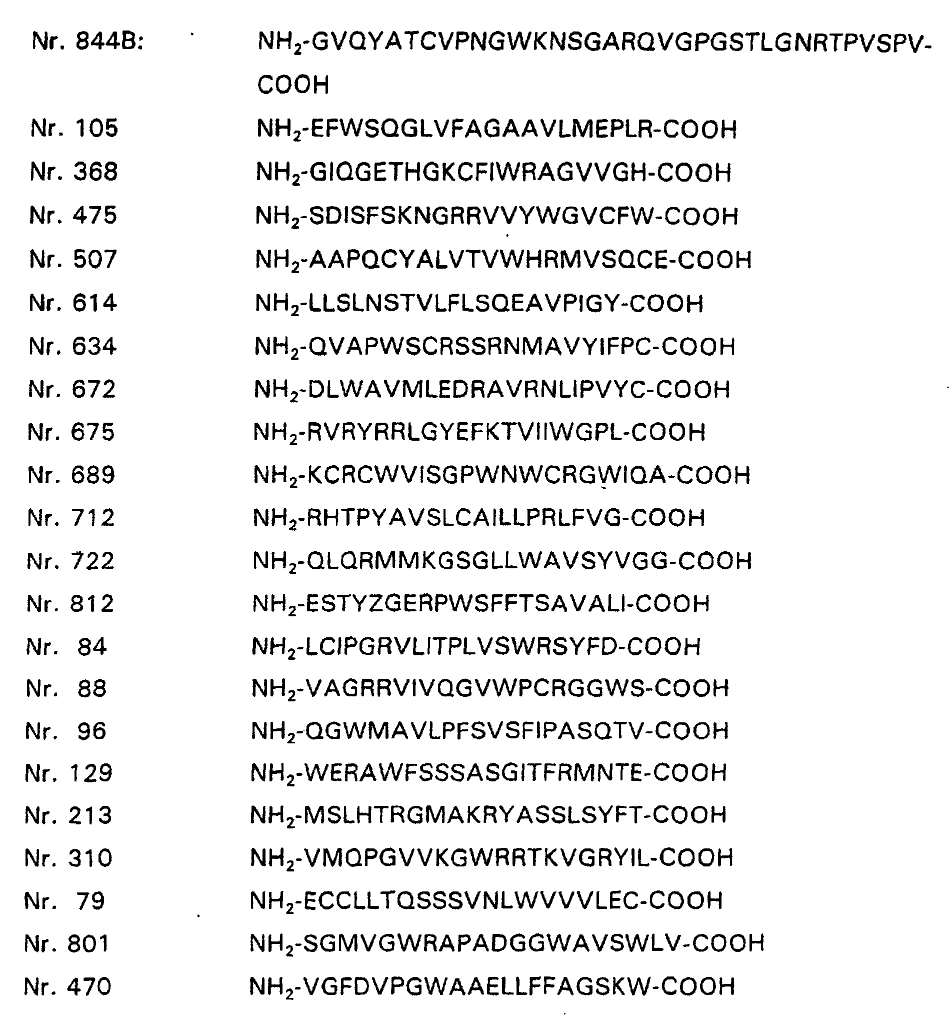

Peptides according to the invention are suited to bind HPV E7 proteins and inhibit the functions thereof.

The expression "HPV E7 proteins" comprises an E7 protein of any HPV type, particularly of HPV1, 5, 6, 11, 16 or 18. An E7 protein may have a wild-type sequence or a sequence differing therefrom by one or several amino acids. In addition, it may be present in a shortened form, i.e. it is only present in the form of the fragment necessary for the binding to a cell regulatory protein. For an E7 protein of HPV16, for example, this is a fragment comprising amino acids 39-98. The fragment can also be present in several copies within the polypeptide molecule. Moreover, an E7 protein and a fragment thereof, respectively, may be present in the form of a fusion protein.

Peptides according to the invention may be present as such or when linked with other substances, e.g. (poly)peptides. The linkage may consist in that the peptides according to the invention are linked with the (poly)peptides via linkers, e.g. disulfide bridges. The peptides according to the invention can also be fused with the (poly)peptides, so that the peptides according to the invention are present in the form of fusion (poly)peptides. For example, leader peptides such as penetratin of Drosophila antennapedia, which support the incorporation of the peptides according to the invention into cells, offer themselves as (poly)peptides for fusion (poly)peptides. On the other hand, polypeptides linked via linkers with the peptide according to the invention may be e.g. carrier proteins, such as transferrin, which are not considered foreign in the body. It is also possible that several peptides according to the invention are simultaneously present when linked with an above-mentioned substance.

A further subject matter of the present invention relates to a nucleic acid, particularly a DNA, which codes for a peptide according to the invention. Such a DNA may be present in vectors, particularly expression vectors. Therefore, they also belong to the subject matter of the present invention.

A further subject matter of the present invention relates to a pharmaceutical composition which contains one or several peptides according to the invention and conventional auxiliary agents. For example, excipients or carriers, binders, blasting agents, lubricating agents, solvents, solvent mediators, release accelerators, release retarders, emulsifiers, stabilizers, etc., can be used as auxiliary agents.

By means of the present invention it is possible to bind HPV E7 proteins and inhibit the function thereof. This serves for achieving that HPV-associated diseases, particularly carcinomas, do not establish. If previously developed, such diseases can also be treated. Moreover, the peptides according to the invention represent a basis for the development of completely new active substances against HPV-associated diseases, particularly carcinomas.

This invention is explained by the below examples.

### Example 1: Expression of a peptide according to the invention in eukaryotic cells

The eukaryotic expression vector pX is used which is controlled by the CMV promoter (Pagano, M. et al., EMBO J. 11, (1992), 961-971). An EcoRI/Sall fragment is ligated thereinto, which comprises the DNA for peptide No. 732 of Table 1 and from which plasmid pJM-1-732 of the peptide library from Colas et al., see above, originates. The expression vector pX-732 is obtained. It codes for a fusion polypeptide from peptide No. 732 and thioredoxin A (TrxA). pX-732 is transfected by means of a calcium phosphate co-precipitation process into the cells, U20S, see above, and the expression of peptide No. 732 is determined by an antibody directed against TrxA.

It shows that a peptide according to the invention can be expressed in cells.

### Example 2: Inhibition of an HPV16 E7 protein by expression of a peptide according to the invention

### (A) Determination of apoptosis

U20S cells, see above, are transfected with 5 µg of the expression vector pX-E7 which codes for an HPV16 E7 protein (Zerfass-Thome, K. et al., Oncogene 13, (1996), 2323-2330). In a parallel batch, the cells are transfected with pX-E7 (5 µg) and pX-732 (1-10 µg) (cf. Example 1).

24 hours after the transfection, the cells are fixed and stained using propidium iodide. The cells are analyzed in a FACscan by measuring the fluorescence caused by propidium iodide. Apoptotic nuclei are shown as nuclei having a DNA content of less than 2N. The amount of apoptotic nuclei is determined in comparison with normal nuclei.

It turns out that the apoptosis amount increases from 5 to 15 % by pX-E7, whereas this effect will not occur if pX-732 is simultaneously present. Thus, a peptide according to the invention inhibits an HPV16 E7 protein as regards its function.

### (B) Determination of cell proliferation

18 after the transfection, the cells from (A) are subjected to 4-hour labeling using bromodeoxyuridine. 18 hours later, the cells are fixed and incubated with an antibody directed against bromodeoxyuridine in combination with an FITC-linked second antibody. The incorporation of bromodeoxyuridine is determined in a FACscan by measuring the FITC-caused fluorescence.

It turns out that the incorporation of bromodeoxyuridine is reduced by 30 % by pX-E7, whereas this effect will not occur if pX-732 is simultaneously present. Thus, a peptide according to the invention inhibits an HPV16 E7 protein as regards its function.

### Example 3: Inhibition of an HPV16 E7 protein by a chemically synthesized peptide according to the invention

(A) Peptide No. 732 is chemically synthesized with a leader attached to the NH₂ end and supporting the incorporation into cells. It is penetratin and originates from Drosophila antennapedia. Thus, peptide No. 732 is available as fusion polypeptide and has the following sequence: NH2-RQIKIWFQNRRMKWKKIAARLRLRCGVILLLCTLDL-COOH
   Cells are used in which HPV16 E7 is expressed and whose growth depends thereon. Such cells are e.g. E7/2 cells. They are transfected with 10 µM - 200 µM of the synthesized peptide No. 732. Growth rate, apoptosis and cell cycle profiles of the cells are determined 1 hour to 72 hours after the transfection. The former is effected by determining the cell number. As regards the second test, reference is made to Example 2 (A). As to the latter test, the cell DNA is stained with propidium iodide and its content is determined in a FACscan. On the basis thereof, the cell cycle profile is assessed by means of the CellQuest program (Schulze, A. et al., Proc. Natl. Acad. Sci, U.S.A. 92, (1995), 11264-11268), the number of cells in the S phase being taken as a marker for the cell proliferation.
   It shows that peptide No. 732 reduces the growth rate, lowers the apoptosis frequency and reduces the number of cells in the S phase. Thus, a peptide according to the invention inhibits an HPV16 E7 protein as regards its function.
(B) In place of penetratin, TAT sequences are linked as "leaders" with peptide No. 732. For this purpose, the BamHI/NdeI fragment (cf. Example 1) of pJM-1 is inserted in the vector pET 19b (Novagen company) so as to obtain the vector pH6-732. Thereafter, cleavage is carried out by means of Ndel and a TAT-cDNA of HIV-1 (Vives, E. et al., J. Biol. Chem. 272, (1997), 16010-16017) is inserted. The expression vector pH6-732-TAT is obtained which codes for an His6-732 fusion polypeptide. After transfection of the E. coli strain BL21(DE3) with this expression vector, the fusion polypeptide is purified via a nickel-agarose column.

The fusion polypeptide is added to the culture medium of E7/2 cells (cf. (A)). Furthermore, the same analyses are carried out as in Example 3 (A).

The results of Example 3 (A) are confirmed.

## Claims

1. A peptide, binding to the C-terminal part of an HPV E7 protein, selected from the following peptides: 
NH2-LKRRCYVQLTREARDPSVQS-COOH
NH2-LNFIFDERSDIYVLWLILEG-COOH
NH2-AVSVVCNTFCKLLGLGGSS-COOH
NH2-VSGGMFQYAAEVLRWGDFYR-COOH
NH2-FGSTSVMVYVEQ-COOH
NH2-PGLAWSWACECCG-COOH
NH2-EHAVASVQEAVTILEG-COOH
NH2-PGHAVSVLFGTFRERAYQST-COOH
NH2-GEWWICFRNLAGSFMGVWLF-COOH
NH2-STSVGNRCQFRPRAVIYEGM-COOH
NH2-VLGTPMLWLMGGVRPYYIGV-COOH
NH2-RNACAVLRVPEPWGEVVPSSGPGVASRELN-COOH
NH2-LVHMFFNEKHVPVCVCTLDL-COOH
NH2-WQSLRRNGSRWLLRWPAEDE-COOH
NH2-IAARLRLRCGVILLLCTLDL-COOH
NH2-GFLQKRVVYSLMFLWSTV-COOH
NH2-GTNTGGDWAVARCWLHENDF-COOH
NH2-GVQYATCVPNGWKNSGARQV-COOH
NH2-GVQYATCVPNGWKNSGARQVGPGSTLGNRTPVSPV-COOH,
NH2-EFWSQGLVFAGAAVLMEPLR-COOH
NH2-GIQGETHGKCFIWRAGVVGH-COOH
NH2-SDISFSKNGRRVVYWGVCFW-COOH
NH2-AAPQCYALVTVWHRMVSQCE-COOH
NH2-LLSLNSTVLFLSQEAVPIGY-COOH
NH2-QVAPWSCRSSRNMAVYIFPC-COOH
NH2-DLWAVMLEDRAVRNLIPVYC-COOH
NH2-RVRYRRLGYEFKTVIIWGPL-COOH
NH2-KCRCWVISGPWNWCRGWIQA-COOH
NH2-RHTPYAVSLCAILLPRLFVG-COOH
NH2-QLQRMMKGSGLLWAVSYVGG-COOH
NH2-ESTYZGERPWSFFTSAVALI-COOH
NH2-LCIPGRVLITPLVSWRSYFD-COOH
 
NH2-VAGRRVIVQGVWPCRGGWS-COOH
NH2-QGWMAVLPFSVSFIPASQTV-COOH
NH2-WERAWFSSSASGITFRMNTE-COOH
NH2-MSLHTRGMAKRYASSLSYFT-COOH
NH2-VMQPGVVKGWRRTKVGRYIL-COOH
NH2-ECCLLTQSSSVNLWVVVLEC-COOH
NH2-SGMVGWRAPADGGWAVSWLV-COOH
NH2-VGFDVPGWAAELLFFAGSKW-COOH
 wherein the peptide may include a sequence modification of up to 40 % and wherein the peptide may be present as a fusion polypeptide.

2. The peptide according to claim 1, wherein the fusion polypeptide comprises a leader sequence.

3. A pharmaceutical composition, comprising one or several peptides according to any one of claims 1 to 2 and conventional auxiliary agents.

4. The pharmaceutical composition according to claim 3, wherein the peptide is present as a fusion polypeptide.

5. The pharmaceutical composition according to claim 4, wherein the fusion polypeptide comprises a leader sequence.

6. Use of the peptide according to any one of claims 1 to 2 to produce a medicament for inhibiting an HPV E7 protein.

7. Use according to claim 6, wherein the inhibition is effected for the prophylaxis and/or treatment of HPV-associated diseases.

8. Use according to claim 7, wherein the diseases comprise carcinoma diseases.

9. Use according to any one of claims 6 to 8, wherein HPV comprises HPV1, HPV5, HPV6, HPV11, HPV16 and HPV18.

## Patentansprüche

1. Peptid, das an den C-terminalen Teil eines HPV E7 Proteins bindet, ausgewählt aus den folgenden Peptiden: 
NH2-LKRRCYVQLTREARDPSVQS-COOH
NH2-LNFIFDERSDIYVLWLILEG-COOH
NH2-AVSVVCNTFCKLLGLGGSS-COOH
NH2-VSGGMFQYAAEVLRWGDFYR-COOH
NH2-FGSTSVMVYVEQ-COOH
NH2-PGLAWSWACECCG-COOH
NH2-EHAVASVQEAVTILEG-COOH
NH2-PGHAVSVLFGTFRERAYQST-COOH
NH2-GEWWICFRNLAGSFMGVWLF-COOH
NH2-STSVGNRCQFRPRAVIYEGM-COOH
NH2-VLGTPMLWLMGGVRPYYIGV-COOH
NH2-RNACAVLRVPEPWGEVVPSSGPGVASRELN-COOH
NH2-LVHMFFNEKHVPVCVCTLDL-COOH
NH2-WQSLRRNGSRWLLRWPAEDE-COOH
NH2-IAARLRLRCGVILLLCTLDL-COOH
NH2-GFLQKRVVYSLMFLWSTV-COOH
NH2-GTNTGGDWAVARCWLHENDF-COOH
NH2-GVQYATCVPNGWKNSGARQV-COOH
NH2-GVQYATCVPNGWKNSGARQVGPGSTLGNRTPVSPV-COOH,
NH2-EFWSQGLVFAGAAVLMEPLR-COOH
NH2-GIQGETHGKCFIWRAGVVGH-COOH
NH2-SDISFSKNGRRVVYWGVCFW-COOH
NH2-AAPQCYALVTVWHRMVSQCE-COOH
NH2-LLSLNSTVLFLSQEAVPIGY-COOH
NH2-QVAPWSCRSSRNMAVYIFPC-COOH
NH2-DLWAVMLEDRAVRNLIPVYC-COOH
NH2-RVRYRRLGYEFKTVIIWGPL-COOH
NH2-KCRCWVISGPWNWCRGWIQA-COOH
NH2-RHTPYAVSLCAILLPRLFVG-COOH
NH2-QLQRMMKGSGLLWAVSYVGG-COOH
NH2-ESTYZGERPWSFFTSAVALI-COOH
NH2-LCIPGRVLITPLVSWRSYFD-COOH
 
NH2-VAGRRVIVQGVWPCRGGWS-COOH
NH2-QGWMAVLPFSVSFIPASQTV-COOH
NH2-WERAWFSSSASGITFRMNTE-COOH
NH2-MSLHTRGMAKRYASSLSYFT-COOH
NH2-VMQPGVVKGWRRTKVGRYIL-COOH
NH2-ECCLLTQSSSVNLWVVVLEC-COOH
NH2-SGMVGWRAPADGGWAVSWLV-COOH
NH2-VGFDVPGWAAELLFFAGSKW-COOH
 wobei das Peptid eine Sequenzmodifikation von bis zu 40 % aufweisen kann und wobei das Peptid als Fusionspolypeptid vorhanden sein kann.

2. Peptid nach Anspruch 1, wobei das Fusionspolypeptid eine Leader-Sequenz umfasst.

3. Pharmazeutische Zusammensetzung, die ein oder mehrere Peptide nach einem der Ansprüche 1 bis 2 und herkömmliche Hilfsstoffe umfasst.

4. Pharmazeutische Zusammensetzung nach Anspruch 3, wobei das Peptid als Fusionspolypeptid vorhanden ist.

5. Pharmazeutische Zusammensetzung nach Anspruch 4, wobei das Fusionspolypeptid eine Leader-Sequenz umfasst.

6. Verwendung des Peptids nach einem der Ansprüche 1 bis 2 zum Herstellen eines Medikaments zur Inhibition eines HPV E7 Proteins.

7. Verwendung nach Anspruch 6, wobei die Inhibition zur Prophylaxe und/oder Behandlung von mit HPV verbundenen Erkrankungen bewirkt wird.

8. Verwendung nach Anspruch 7, wobei die Erkrankungen Krebserkrankungen umfassen.

9. Verwendung nach einem der Ansprüche 6 bis 8, wobei HPV HPV1, HPV5, HPV6, HPV11, HPV16 und HPV18 umfasst.

## Revendications

1. Peptide, se liant à la partie C-terminale d'une protéine E7 de HPV, choisi parmi les peptides suivants : 
NH2-LKRRCYVQLTREARDPSVQS-COOH
NH2-LNFIFDERSDIYVLWLILEG-COOH
NH2-AVSVVCNTFCKLLGLGGSS-COOH
NH2-VSGGMFQYAAEVLRWGDFYR-COOH
NH2-FGSTSVMVYVEQ-COOH
NH2-PGLAWSWACECCG-COOH
NH2-EHAVASVQEAVTILEG-COOH
NH2-PGHAVSVLFGTFRERAYQST-COOH
NH2-GEWWICFRNLAGSFMGVWLF-COOH
NH2-STSVGNRCQFRPRAVIYEGM-COOH
NH2-VLGTPMLWLMGGVRPYYIGV-COOH
NH2-RNACAVLRVPEPWGEVVPSSGPGVASRELN-COOH
NH2-LVHMFFNEKHVPVCVCTLDL-COOH
NH2-WQSLRRNGSRWLLRWPAEDE-COOH
NH2-IAARLRLRCGVILLLCTLDL-COOH
NH2-GFLQKRVVYSLMFLWSTV-COOH
NH2-GTNTGGDWAVARCWLHENDF-COOH
NH2-GVQYATCVPNGWKNSGARQV-COOH
NH2-GVQYATCVPNGWKNSGARQVGPGSTLGNRTPVSPV-COOH,
NH2-EFWSQGLVFAGAAVLMEPLR-COOH
NH2-GIQGETHGKCFIWRAGVVGH-COOH
NH2-SDISFSKNGRRVVYWGVCFW-COOH
NH2-AAPQCYALVTVWHRMVSQCE-COOH
NH2-LLSLNSTVLFLSQEAVPIGY-COOH
NH2-QVAPWSCRSSRNMAVYIFPC-COOH
NH2-DLWAVMLEDRAVRNLIPVYC-COOH
NH2-RVRYRRLGYEFKTVIIWGPL-COOH
NH2-KCRCWVISGPWNWCRGWIQA-COOH
NH2-RHTPYAVSLCAILLPRLFVG-COOH
NH2-QLQRMMKGSGLLWAVSYVGG-COOH
NH2-ESTYZGERPWSFFTSAVALI-COOH
NH2-LCIPGRVLITPLVSWRSYFD-COOH
 
NH2-VAGRRVIVQGVWPCRGGWS-COOH
NH2-QGWMAVLPFSVSFIPASQTV-COOH
NH2-WERAWFSSSASGITFRMNTE-COOH
NH2-MSLHTRGMAKRYASSLSYFT-COOH
NH2-VMQPGVVKGWRRTKVGRYIL-COOH
NH2-ECCLLTQSSSVNLWVVVLEC-COOH
NH2-SGMVGWRAPADGGWAVSWLV-COOH
NH2-VGFDVPGWAAELLFFAGSKW-COOH
 ledit peptide pouvant comprendre une modification de séquence en une proportion allant jusqu'à 40 %, et ledit peptide pouvant être présent sous forme d'un polypeptide de fusion.

2. Peptide suivant la revendication 1, dans lequel le polypeptide de fusion comprend une séquence leader.

3. Composition pharmaceutique, comprenant un ou plusieurs peptides suivant l'une quelconque des revendications 1 et 2 et des agents auxiliaires classiques.

4. Composition pharmaceutique suivant la revendication 3, dans laquelle le peptide est présent sous forme d'un polypeptide de fusion.

5. Composition pharmaceutique suivant la revendication 4, dans laquelle le polypeptide de fusion comprend une séquence leader.

6. Utilisation du peptide suivant l'une quelconque des revendications 1 et 2 pour la production d'un médicament destiné à inhiber une protéine E7 de HPV.

7. Utilisation suivant la revendication 6, dans laquelle l'inhibition est effectuée pour la prophylaxie et/ou le traitement de maladies associées au HPV.

8. Utilisation suivant la revendication 7, dans laquelle les maladies comprennent des maladies cancéreuses.

9. Utilisation suivant l'une quelconque des revendications 6 à 8, dans laquelle le HPV comprend HPV1, HPV5, HPV6, HPV11, HPV16 et HPV18.
